# EUROPEAN PATENT SPECIFICATION

(11) **EP 1 954 339 B1**
(45) Date of publication and mention of the grant of the patent: **29.08.2012**
(21) Application number: 06838576.4
(22) Date of filing: 29.11.2006
(51) Int. Cl.: A61M 25/01, A61J 15/00

(54) **SHORT WIRE PEG AND PEG-J TUBE**
KURZER DRAHTSTIFT UND STIFT-J-ROHR
SONDE PEG ET PEG-J A FIL COURT

(30) Priority: 30.11.2005 US 740701 P
(43) Date of publication of application: 13.08.2008
(73) Proprietor: Cook Medical Technologies LLC, Bloomington, IN 47404 (US)
(72) Inventor: DEAL, Stephen, E., Charlotte, NC 28211 (US)
(74) Representative: Garratt, Peter Douglas
(86) International application number: PCT/US2006/045690
(87) International publication number: WO 2007/064705

(56) References cited:
- US-A- 4 573 576
- US-A- 4 824 435
- US-A- 4 826 481
- US-A- 5 334 167

## Description

### RELATED APPLICATION

This application claims the benefit of priority from U.S. Provisional Application No. 60/740,701, filed November 30, 2005, and entitled "Short Wire Peg and Peg-J Tube"

### TECHNICAL FIELD

The present invention relates to medical devices, and more particularly to feeding tubes for delivery of nutritional products to the intestinal tract and methods for placement of feeding tubes therein.

### BACKGROUND OF THE INVENTION

Patients for which normal ingestion of food becomes difficult or impossible may require placement of a feeding tube to assist in providing their nutritional needs. For some individuals, such as comatose patients, stroke victims, or those with a compromised gastrointestinal tract, this may require placement of a tube that is introduced percutaneously into the stomach for delivery of nutritional products directly into the stomach or jejunum. The procedure, known as a Percutaneous Endoscopic Gastrostomy (PEG), involves the introduction of an endoscope into the stomach. The desired site where the stoma is to be created is indicated from above by depressing the abdomen and viewing the depressed site with the endoscope. Transillumination may also be utilized to locate the desired site through the abdominal wall. A sheathed needle or trocar punctures the abdominal wall and enters the stomach, creating a stoma. The needle is removed and a looped insertion wire is introduced through the sheath where it is grasped by a snare or forceps deployed from the working channel of the endoscope. Once it is captured, the insertion wire is pulled into the working channel of the endoscope. The endoscope is then withdrawn from the patient via the oral cavity, pulling the insertion wire with it.

In the standard Ponsky method (or "pull" method), the distal loop of a percutaneous gastrostomy feeding tube is coupled to the insertion wire loop exiting the patient's mouth. With the insertion wire now tethered to the gastrostomy feeding tube, the endoscopist retracts the portion of the insertion wire exiting the stoma, thereby pulling the gastrostomy feeding tube into the patient's mouth and towards the stomach. With continued retraction of the insertion wire, the distal end of the gastrostomy feeding tube is pulled out through the stoma. The gastrostomy feeding tube typically includes a tapered dilator portion to aid its passage through the stoma. Once the feeding tube has been properly positioned with the proximal end cap or bolster of the feeding tube against the internal wall of the stomach, it is secured by an external bolster positioned against the outside of the abdomen wall. The endoscope is typically reintroduced into the stomach to check the internal position of the feeding tube before the external bolster is secured. The dilator portion of the feeding tube is then cut away and removed and an adapter compatible with standardized feeding pumps and syringes is attached.

Reference is directed to US 4 573 576 in which there is disclosed a percutaneous gastrostomy kit which comprises a mushroom catheter with a transverse bore at a closed proximal end and an enlarged washer on the distal end adjacent the mushroom head. A length of suture thread can be secured to the proximal end of the catheter by passing the suture through the transverse bore to enable to a catheter to be pulled through the gastrointestinal tract.

In a variation of the PEG procedure know as the "push" method, the gastrostomy feeding tube is advanced or pushed down the esophagus by the physician and into position in the stomach using a wire guide that has been placed in the same manner as the insertion wire in the "pull" method. More specifically, the feeding tube is loaded onto the portion of the wire guide exiting the patient's mouth by passing the end of the wire guide through a lumen extending through the length of the feeding tube. While holding the wire guide stationary, the physician pushes the feeding tube along the wire guide through the patient's mouth, into the stomach, and then out through the stoma. The feeding tube is then secured in the same manner as in the "pull" method.

The "push" method was developed to overcome certain problems associated with the "pull" method, such as complications associated with the pulling of the gastrostomy feeding tube through the esophagus. For example, the direction of the retraction force applied to the feeding tube by the insertion wire during the "pull" method tends to pull the distal end of the feeding tube against the wall of the esophagus thereby irritating or injuring the esophagus as it pulled therethough. The "push" method reduces this problem because the distal end of the feeding tube tends to follow the path of least resistance through the esophagus, and is not pulled to one side or the other by the insertion wire. In addition, movement of the insertion wire as it is pulled through the stoma during the "pull" method, particularly since the insertion wire is under traction, may irritate or injure the stoma. In the "push" method, the wire guide is held stationary during insertion and placement of the feeding tube and therefore the likelihood of irritation or injury caused by the wire guide is greatly reduced. There is also the possibility that the insertion wire may separate from the feeding tube during the "pull" method of insertion, thereby necessitating that the procedure be started over with the placement of a new insertion wire. In contrast, the feeding tube is not tied to the end of the wire guide in the "push" method and therefore does not depend upon the interconnection of these two components.

Irrespective of the above, the "push" method has certain disadvantages. For example, the wire guide utilized during the procedure must be relatively long. This is because the portion of the wire guide exiting out of the patient's mouth must be sufficiently long to pass completely through gastrostomy feeding tube so that the physician can grasp and hold the wire guide stationary while pushing the feeding tube into the patient. Likewise, the portion of the wire guide exiting out of the stoma is preferably long enough to extend past the distal end of the feeding tube as the feeding exits the stoma, particularly if the wire guide is needed for re-positioning of the feeding tube or other procedures. Thus, the wire guide may need to have a length that is at least twice as long as the feeding tube, plus the length of the internal pathway extending between the patient's mouth and the stoma. For example, a 300 cm wire guide may be required to introduce a 100 cm long feeding tube using the "push" method. Wire guides of this length are unwieldy and easily contaminated.

What is needed is gastrostomy feeding tube that can be introduced using the "push" method with a much shorter wire guide. Preferably, the wire guide should not be significantly longer than the overall length of the feeding tube or the length of the pathway extending between the patient's mouth and the stoma. The present invention meets this need by providing a gastrostomy feeding tube that can be introduced using the "push" method with a much shorter wire guide.

In another variation of the PEG procedure, a jejunostomy (jejunal) feeding tube is coupled to the gastrostomy feeding tube and inserted into the small bowel of the patient. Intrajejunal tube feeding is used to deliver nutritional products directly into the jejunum, and is typically employed for patients with gastroparesis or those at a high risk for gastric-feeding aspiration. In this procedure, known as Percutaneous Endoscopic Jejunostomy (PEJ or PEG-J), an endoscope is first introduced (or reintroduced) into the stomach. The distal end of a wire guide is then passed through the gastrostomy feeding tube and into the stomach, whereby it is grasped by a snare or forceps deployed from the working channel of the endoscope. Once it is captured, the snare or forceps is used to push the distal end of the wire guide into the small bowel as far as possible. The snare or forceps is then withdrawn from the small bowel so that it will not interfere with the placement of the jejunostomy feeding tube therein. The jejunostomy feeding tube is loaded onto the portion of the wire guide exiting the gastrostomy tube by passing the end of the wire guide through a lumen extending through the length of the jejunostomy feeding tube. While holding the wire guide stationary, the physician pushes the jejunostomy feeding tube along the wire guide and through the gastrostomy feeding tube, into the stomach, and then into the small bowel. The proximal end of the jejunostomy feeding tube is then secured to the gastrostomy feeding tube and an adapter compatible with standardized feeding pumps and syringes is attached. Once the jejunostomy feeding tube is in position, the endoscope is withdrawn.

Placement of the jejunostomy feeding tube by introduction over a wire guide by the above-described method has certain disadvantages. For example, the wire guide utilized during the procedure must be relatively long. This is because the portion of the wire guide exiting out of the gastrostomy feeding tube must be sufficiently long to extend completely through the jejunostomy feeding tube so that the physician can grasp and hold the wire guide stationary while pushing the jejunostomy feeding tube into the patient.

What is needed is jejunostomy feeding tube that can be introduced using the above-described method with a much shorter wire guide. Preferably, the wire guide should not be significantly longer than the overall length of the jejunostomy feeding tube. The present invention meets this need by providing a jejunostomy feeding tube that can be introduced using the above-described method with a much shorter wire guide.

### SUMMARY OF THE INVENTION

The foregoing problems are solved and a technical advance is achieved with an illustrative feeding tube , whereby the feeding tube is introduced over a wire guide of reduced length as further disclosed in claim 1. The feeding tube comprises an elongate shaft having a distal end and proximal end. The feeding tube comprises a coupling region for coupling the feeding tube to a wire guide. The coupling region is disposed near the distal end of the elongate shaft and has a length that is substantially less than the length of the elongate shaft. The feeding tube is introduced within the patient by coupling the coupling region of the feeding tube to a previously introduced wire guide, and then pushing the feeding tube distally along the wire guide. The length of the portion of the previously introduced wire guide exiting the patient needs to be only slightly longer than the length of the coupling region to enable the user to maintain control of the wire during coupling and introduction of the feeding tube. Depending on the length of the coupling region, the overall length of the wire guide need not be significantly longer than the overall length of the feeding tube or the length of the internal passageway extending between the patient's mouth and the stoma.

The coupling region comprises a lumen extending through at least a portion of the elongate shaft. The lumen is sized to permit a wire guide to pass therethrough and extends between a distal port near the distal end of the shaft and a proximal port spaced distally from the proximal end of the shaft. In an illustrative embodiment of the invention, the distal port is disposed in the distal end of the shaft and the proximal port extends through a side wall of the shaft, wherein the proximal port is located a substantially greater distance from the proximal end of the shaft than from the distal end of the shaft. The lumen may be coincident with the feeding lumen of the feeding tube, or may be a separate wire guide lumen that is spaced apart from the feeding lumen. The lumen may also be formed by a separate wire guide tube attached to the side wall of the shaft.

In another aspect of the invention, the feeding tube comprises a short wire gastrostomy (or PEG) feeding tube that can be introduced within the patient with a relatively short wire guide using a variation of the "push" method of introduction. The short wire PEG tube comprises an elongate shaft having a distal end and proximal end. A bolster is affixed to the proximal end of the shaft and is configured to engage the interior surface of the stomach wall. An exterior bolster is provided separately and is configured to attach to the shaft and engage the exterior surface of the abdomen. The proximal portion of the shaft comprises a feeding tube having a feeding lumen disposed therethrough. The distal portion of the shaft comprises a removable lead catheter that is preferably tapered to facilitate ingress through the gastrointestinal tract of the patient. A distal port is disposed in the distal end of the lead catheter and a proximal port is disposed through the side wall of the lead catheter, the proximal port being located a relatively short distance proximally from the distal port and a substantially greater distance distally from the proximal end of the shaft. In one embodiment of the invention, the proximal port is spaced approximately 5-10 cm from the distal port. A lumen extends between the distal port and the proximal port, and is sized to accommodate a wire guide therethrough. The lumen forms a coupling region for coupling the short wire PEG tube to the wire guide and has a length that is substantially less than the overall length of the shaft.

The short wire PEG tube is introduced within the patient by passing an end of a previously placed wire guide in through the distal port, through the lumen, and out through proximal port. The portion of the wire guide extending out through the proximal port is then grasped and held stationary as the short wire PEG tube is advanced along the wire guide and into the patient using a variation of the "push" method of introduction. Because the short wire PEG tube is only coupled to the wire guide for a relatively short length, as compared to the overall length of the shaft, the length of wire guide extending out of the patient is significantly less than the overall length of the short wire PEG tube. This permits the use of a much shorter wire guide than would be required for the introduction of a standard PEG tube using the "push" method. Other aspects of the procedure for the final positioning and placement of the short wire PEG tube are similar to the procedure used for final positioning and placement of a standard PEG tube.

In another aspect of the invention, the feeding tube comprises a short wire jejunostomy (or PEG-J) feeding tube that can be introduced within the patient with a relatively short wire guide. The short wire PEG-J tube comprises an elongate shaft having a distal end and proximal end. A connector fitting is affixed to the proximal end of the shaft and is configured to engage the feeding lumen of a PEG tube previously placed though the stomach and abdominal walls of a patient. The shaft comprises a feeding lumen disposed therethrough. A distal port is disposed in the distal end of the shaft and a proximal port is disposed through the side wall of the shaft, the proximal port being located a relatively short distance proximally from the distal port and a substantially greater distance distally from the proximal end of the shaft. In one embodiment of the invention, the proximal port is spaced approximately 5-10 cm from the distal port. A lumen extends between the distal port and the proximal port, and is sized to accommodate a wire guide therethrough. In one embodiment of the invention, the lumen extending between the distal and proximal ports comprises a portion of the feeding lumen. The lumen forms a coupling region for coupling the short wire PEG-J tube to the wire guide and has a length that is substantially less than the overall length of the shaft.

The short wire PEG-J tube is introduced within the patient by passing an end of a previously placed wire guide in through the distal port, through the lumen, and out through proximal port. The portion of the wire guide extending out though the proximal port is then grasped and held stationary as the short wire PEG-J tube is advanced along the wire guide and into the patient through a previously placed PEG tube. Because the short wire PEG-J tube is only coupled to the wire guide for a relatively short length, as compared to the overall length of the shaft, the length of wire guide extending out of the patient is significantly less than the overall length of the short wire PEG-J tube. This permits the use of a much shorter wire guide than would be required for the introduction of a standard PEG-J tube. Other aspects of the procedure for the final positioning and placement of the short wire PEG-J tube are similar to the procedure used for final positioning and placement of a standard PEG-J tube.

These and other advantages, as well as the invention itself, will become apparent in the details of construction and operation as more fully described below. Moreover, it should be appreciated that several aspects of the invention can be used with other types of stent delivery catheters or medical devices.

### BRIEF DESCRIPTION OF THE DRAWINGS

FIG. 1 is a side view of one embodiment of the short wire feeding tube of the present invention, and in particular a short wire gastrostomy (or PEG) feeding tube.

FIG. 2 is a side view of another embodiment of the short wire feeding tube of the present invention, and in particular a short wire jejunostomy (or PEG-J) feeding tube.

FIGS. 3-7 illustrate successive steps in an exemplary method of introducing and placing a short wire gastrostomy (or PEG) feeding tube into the gastric lumen of a patient.

FIGS. 8-9 illustrate successive steps in an exemplary method of introducing and placing a short wire jejunostomy (or PEG-J) feeding tube into the gastric lumen of a patient.

### DESCRIPTION OF THE INVENTION

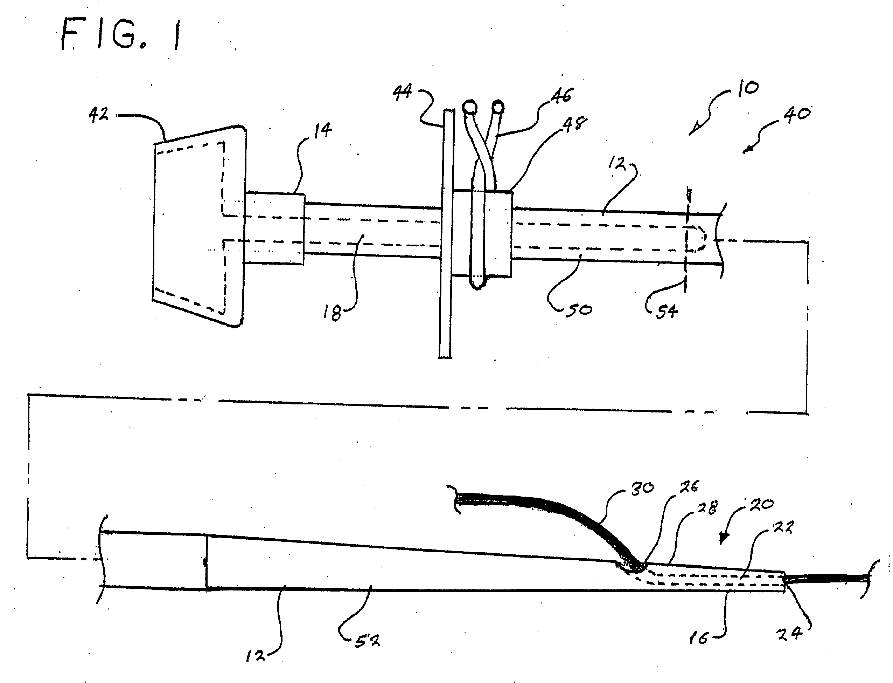

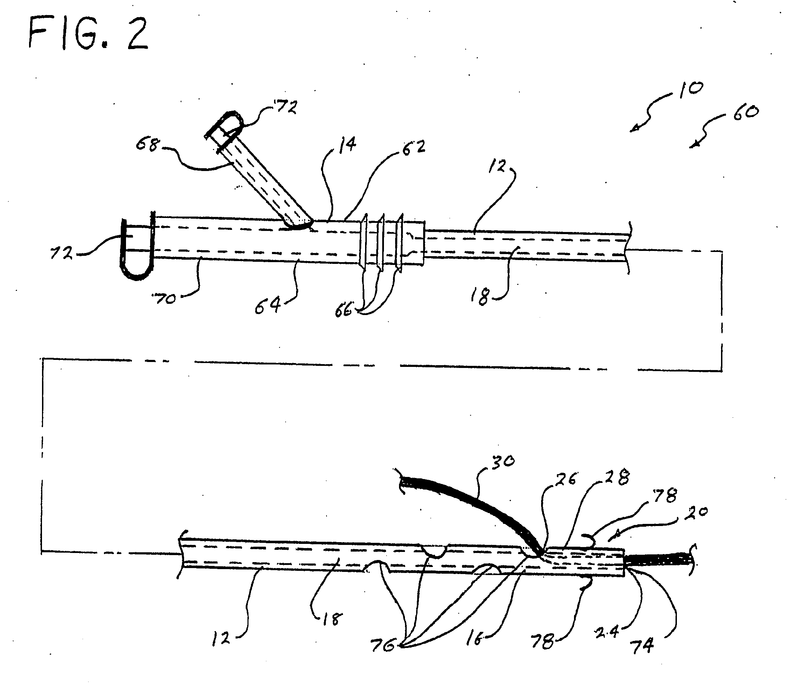

Various embodiments of the short wire feeding tube 10 according to the present invention are shown in Figs. 1-2. In general, the short wire feeding tube 10 comprises an elongate shaft 12 having a proximal end 14 and a distal end 16. A feeding lumen 18 extends through at least a portion of the shaft 12 and provides a passageway for the delivery of nutritional products or medications directly into the stomach or jejunum of the patient. As will be explained in greater detail below, one embodiment of a short wire feeding tube according to the present invention is a short wire gastrostomy (or PEG) feeding tube 40 (shown in Fig. 1) that is positioned through the stomach and abdominal walls of the patient. The short wire PEG feeding tube 40 allows the delivery of nutritional products or medications directly into the patient's stomach. Another embodiment of a short wire feeding tube according to the present invention is a jejunostomy (or PEG-J) feeding tube 60 (shown in Fig. 2) that is positioned between a previously placed PEG tube and the jejunum of the patient. The short wire PEG-J tube 60 allows the delivery of nutritional products or medications directly into the patient's jejunum.

The short wire feeding tube comprises a coupling region 20 for coupling the feeding tube 10 to a wire guide 30. The coupling region 20 is disposed near the distal end 16 of the elongate shaft 12 and has a length that is substantially less than the length of the elongate shaft 12. As will be explained in greater detail below, the feeding tube 10 is introduced and into and placed within the patient by coupling the coupling region 20 of the feeding tube 10 to a previously introduced wire guide 30, and then pushing the feeding tube 10 distally along the wire guide 30 and into the patient. The length of the portion of the previously introduced wire guide 30 exiting the patient, and to which the feeding tube 10 is coupled, needs to be only slightly longer than the length of the coupling region 20 to enable the user to maintain control of the wire guide during the step of coupling and introducing the feeding tube 10. Depending on the length of the coupling region 20, the overall length of the wire guide 30 need not be significantly longer than the overall length of the feeding tube 10, and may actually be shorter than the overall length of the feeding tube 10.

In the embodiments illustrated in Figs. 1-2, the coupling region 20 comprises a wire guide lumen 22 extending through at least a portion of the elongate shaft 12. The wire guide lumen 22 is sized to permit a wire guide 30 to pass therethrough and extends between a distal port 24 near the distal end 16 of the shaft 12 and a proximal port 26 spaced distally from the proximal end 14 of the shaft 12. In the particular embodiment illustrated, the distal port 24 is disposed in the distal end 16 of the shaft 12 and the proximal port 26 extends through a side wall 28 of the shaft 12, wherein the proximal port 26 is located a substantially greater distance from the proximal end 14 of the shaft than from the distal end 16 of the shaft 12.

As shown in Fig. 2, the wire guide lumen 22 may be coincident with the feeding lumen 18 of the feeding tube 10. In other words, the wire guide lumen 22 may merely comprise a portion of the feeding lumen 18. In the alternative, and as shown in Fig. 1, the wire guide lumen 22 may be separate and spaced apart from the feeding lumen 18. In yet another alternative embodiment, the wire guide lumen 22 may be formed by a separate wire guide tube (not shown) attached to the side wall 28 of the shaft 12. Other mechanisms for coupling the feeding tube 10 to the wire guide 30 include one or more clips or loops attached to the shaft 12 and through which the wire guide 30 passes.

In the embodiment illustrated in Fig. 1, the feeding tube comprises a short wire gastrostomy (or PEG) feeding tube 40 that can be introduced within the patient with a relatively short wire guide 30 using a variation of the "push" method of introduction. The short wire PEG tube 40 comprises an elongate shaft 12 having a distal end 16 and a proximal end 14. An internal bolster (or bumper) 42 is affixed to the proximal end 14 of the shaft 12 and is configured to engage the interior surface of the stomach wall. In the particular embodiment illustrated, the overall length of the shaft 12 is between 105 and 145 cm. An exterior bolster 44 is provided and is configured to attach to the shaft 12 so as to engage the exterior surface of the abdomen. In the embodiment illustrated, the external bolster 44 is secured to the shaft 12 by a spring clamp 46 that presses the sleeve 48 of the external bolster 44 into frictional engagement with the exterior surface of the shaft 12.

The proximal portion of the shaft 12 comprises a feeding catheter 50 having a feeding lumen 18 disposed therethrough. In the particular embodiment illustrated, the feeding catheter 50 comprises an 18-24 French tube having a 4-5.5 mm diameter feeding lumen 18. The distal portion of the shaft 12 comprises a removable lead catheter 52 that is preferably tapered to facilitate ingress through the gastrointestinal tract of the patient. As will be explained in greater detail below, the lead catheter 52 is removed from the feeding catheter 50 by, for example, severing the shaft 12 along cut line 54. A distal port 24 is disposed in the distal end 16 of the lead catheter 52 and a proximal port 26 is disposed through the side wall 28 of the lead catheter 52, the proximal port 26 being located a relatively short distance proximally from the distal port 24 and a substantially greater distance distally from the proximal end 14 of the shaft 12. In the embodiment illustrated, the proximal port 26 is spaced approximately 5-10 cm from the distal port 24. A wire guide lumen 22 extends between the distal port 24 and the proximal port 26, and is sized to accommodate a wire guide 30 therethrough. The wire guide lumen 22 forms a coupling region 20 for coupling the short wire PEG tube 40 to the wire guide 30 and has a length that is substantially less than the overall length of the shaft 12.

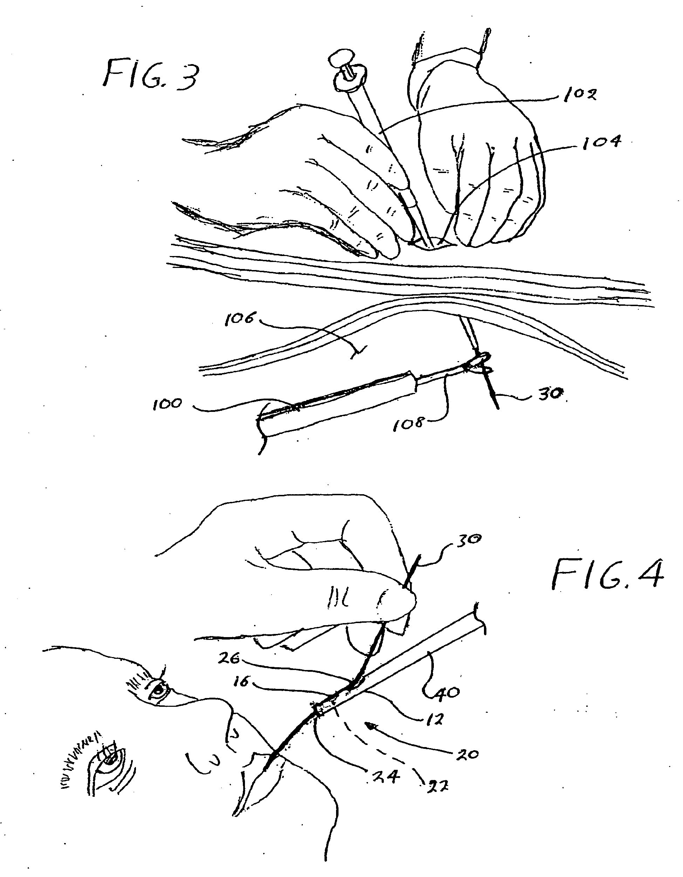

The short wire PEG tube 40 is introduced within the patient using a variation of the standard "push" method of Percutaneous Endoscopic Gastrostomy (PEG). An exemplary embodiment of this procedure is illustrated in Figs. 3-7. With reference to Fig. 3, an endoscope 100 is introduced through the mouth and into the stomach of the patient. A standard Esophagogastroduodenoscopy (EGD) is then typically performed with the endoscope. An EGD is an examination of the lining of the esophagus, stomach, and upper duodenum with the endoscope's camera. The endoscope is then used to identify the desired site where the stoma is to be created. The site is typically identified by depressing the abdomen from outside the patient. Transillumination may also be utilized to locate the desired site through the abdominal wall. For example, the room lights are dimmed so that the light emitted from the end of the endoscope can be seen from outside the patient (i.e., through the stomach and abdominal wall tissue). Once the target site has been identified, a trocar or sheathed needle 102 punctures the abdominal and stomach walls to create a stoma 104, with the distal end of needle 102 entering the interior of the stomach cavity 106. The needle 102 is removed and a wire guide 30 is introduced through the sheath and into the stomach cavity 106. The distal end of the wire guide 30 is then grasped by grasping device 108, such as a snare or forceps, which is deployed from the working channel of the endoscope 100. Once end of the wire guide 30 is captured, the grasping device 108 is retracted so as to pull distal end of the wire guide 30 into the working channel of the endoscope 100. The endoscope 100 is then withdrawn from the patient via the oral cavity, pulling the distal end of the wire guide 30 with it.

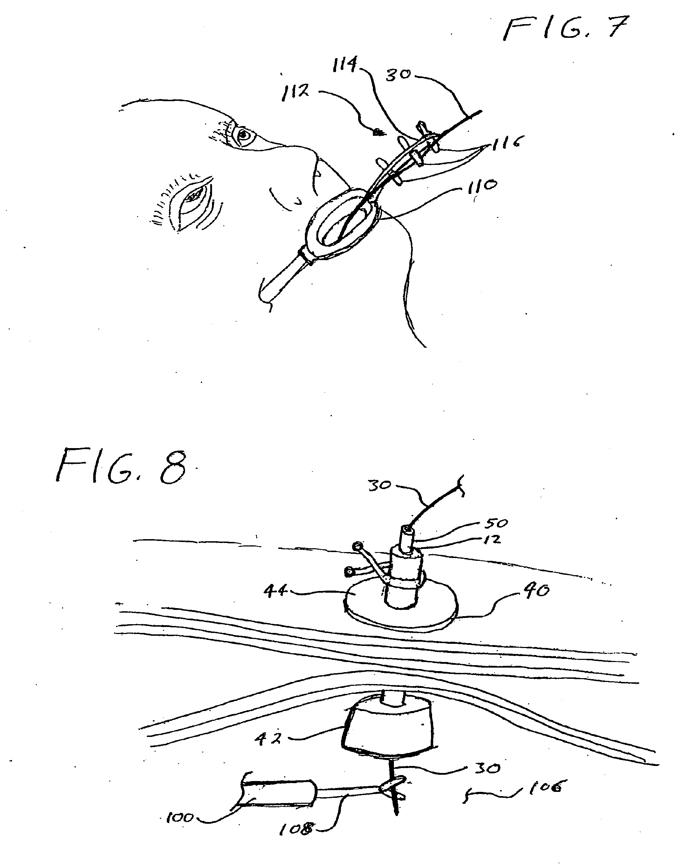

If desired, the distal end of the wire guide 30 may be secured in a bite block having a wire locking mechanism to prevent inadvertent or accidental movement of the wire guide 30. Fig. 7 illustrates an example of a bite block 110 having wire locking mechanism 112, wherein the wire locking mechanism 112 comprises an outwardly projecting spine 114 with a plurality of transversely oriented pins 116 spaced there along. The distal end of the wire guide 30 is secured to the locking mechanism 112 by weaving the wire guide 30 over and under two or more of the pins 116. The pins 116 bend the wire guide 30 so as to create a friction force therebetween that is sufficient to prevent unintended movement between these components.

With reference to Fig. 4, the short wire PEG tube 40 is introduced within the patient by first coupling the distal end of the feeding tube shaft 12 to the distal end of the wire guide 30. In particular, the portion of the wire guide 30 extending out from the patient's mouth is inserted in through the distal port 24, along the wire guide lumen 22, and out through proximal port 26 of the feeding tube's coupling region 20. The portion of the wire guide 30 (i.e., the distal end) extending out through the proximal port 26 is then grasped and held stationary as the short wire PEG tube 40 is advanced along the wire guide 30 and into the patient using a variation of the "push" method of introduction. If the wire guide 30 has been secured in a bite block 110 as described above, the wire guide 30 must be disconnected from the bite block locking mechanism 112 to allow the coupling region 22 to be advanced past the locking mechanism 112. Once the coupling region 22 has been advanced past the bite block 110, the distal end of the wire guide 30 can be re-secured to the locking mechanism 112 to prevent inadvertent or accidental movement thereof.

Because the short wire PEG tube 40 is only coupled to the wire guide 30 for a relatively short length, as compared to the overall length of the shaft 12, the length of wire guide 30 extending out of the patient may be significantly shorter than the overall length of the short wire PEG tube 40. More specifically, the portion of the wire guide 30 extending out of the patient's mouth only needs to be slightly longer than the length of the feeding tube coupling region 22 to allow the physician to maintain control of the wire guide 30 as the short wire PEG tube 40 is being coupled thereto. For example, if the feeding tube coupling region 22 is 6 cm in length, then the length of wire guide 30 extending out of the patient's mouth could be as short as 10 cm. As a consequence, a much shorter wire guide 30 can be utilized than would be required for the introduction of a standard PEG tube having a wire guide lumen extending through the entire length thereof and using the typical "push" method of introduction. However, because the wire guide 30 does not pass through a substantial portion of the shaft 12 for the short wire PEG tube 40, the shaft of the feeding tube must be sufficiently stiff to allow the short wire PEG tube 40 to be pushed along the wire guide 30 and through the gastric lumen without buckling or bending excessively.

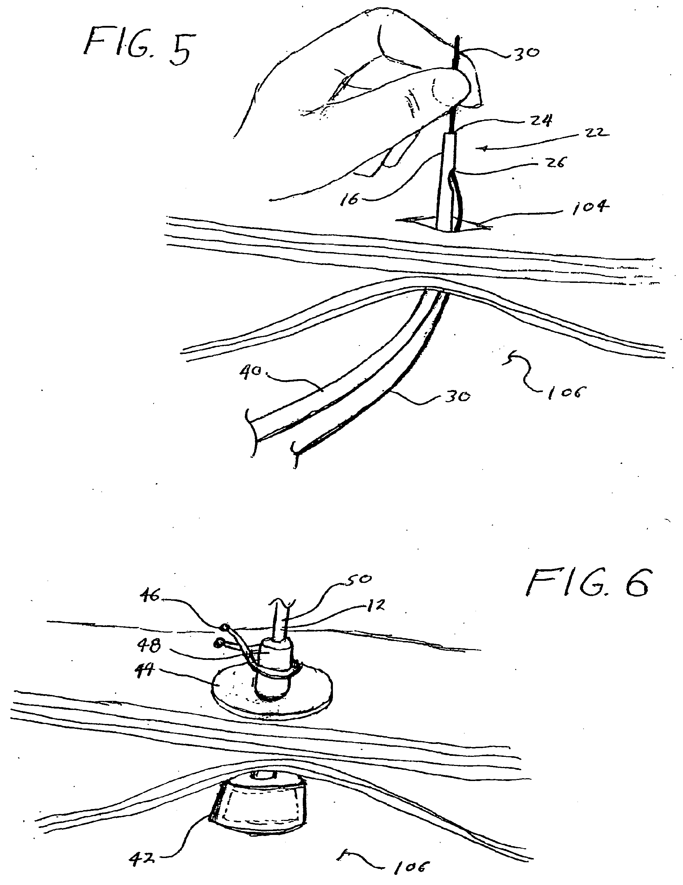

With continued advancement of the short wire PEG tube 40, the distal end 16 of the shaft 12 (i.e., the feeding tube coupling region 22) is pushed along the wire guide 30 and into the patient's stomach 106. As illustrated in Fig. 5, further advancement of the short wire PEG tube 40 causes the distal end 16 of the shaft 12 to be pushed out through the stoma 104. As noted above, the lead catheter portion 52 of the shaft 12 preferably includes a tapered dilator portion (see Fig, 1) to aid its passage through the stoma 104. Once the lead catheter portion 52 of the shaft 12 passes out through the stoma 104, the wire guide 30 is pulled out through the stoma 104 and removed (disengaged) from the feeding tube coupling region 22. The endoscope 100 may then be reintroduced into the stomach cavity 106 to check the internal position of the short wire PEG tube 104 (i.e., the position of the internal bolster 42). As illustrated in Fig. 6, once the short wire PEG tube 40 has been properly positioned with the internal bolster 42 of the feeding tube against the internal wall of the stomach, it is secured by an external bolster 44 secured to the shaft 12 and positioned against the outside of the abdomen wall. The lead catheter 52 (dilator portion) of the shaft 12 is then cut away and removed and an adapter compatible with standardized feeding pumps and syringes is attached. Other aspects of the procedure for the introduction and placement of the short wire PEG tube 40 not described herein are similar to the standard "push" procedure for introduction and placement of a standard PEG tubes and have not been repeated here.

In the embodiment illustrated in Fig. 2, the feeding tube comprises a short wire jejunostomy (or PEG-J) feeding tube 60 that can be introduced within the patient with a relatively short wire guide 30. The short wire PEG-J tube 60 comprises an elongate shaft 12 having a distal end 16 and a proximal end 14. In the particular embodiment illustrated, the shaft 12 is 9-12 French and approximately 60 cm in length. A connector fitting 62 is affixed to the proximal end 14 of the shaft 12 and is configured to engage the feeding lumen of a PEG tube previously placed though the stomach and abdominal walls of a patient. More specifically, the connector fitting 62 is configured to mate with the PEG tube so as to form a seal between the exterior surface 64 of the connector fitting 62 and the internal surface of the PEG tube feeding lumen. The connecter fitting 62 may include a sealing mechanism 66 to secure the two components together and improve the seal therebetween. This arrangement prevents air, fluids, or other stomach contents from passing or leaking out through the juncture of these two components. In the particular embodiment illustrated, the connector fitting 62 comprises a first (or "G") tube 68 and a second (or "J") tube 70, each with a removable sealing cap 72 attached to the ends thereof. The first and second tubes 68, 70 permit the delivery of nutritional products or medications, and/or gastric drainage or decompression. The first and second tubes 68, 70 may be connected (i.e., in fluid communication with) to a common lumen (e.g., feeding lumen 18), or may be connected to separate lumens.

The shaft 12 of the short wire PEG-J tube 60 comprises a feeding lumen 18 disposed therethrough. The feeding lumen 18 typically extends through entire length of the shaft 12, and is configured for the passage of nutritional products or medications from the proximal end 14 of the shaft 12 (i.e., through the connector fitting 62) to the distal end 16 of the shaft 12. A distal feeding port 74 is disposed in the distal end 16 of the shaft 12 and is in communication with the feeding lumen 18 to allow nutritional products or medications passing through the feeding lumen 18 to exit the short wire PEG-J feeding tube 60. The shaft 12 may also include additional feeding openings 76 through the side wall 28 of the shaft 12 and spaced along the distal portion thereof. The feeding openings 76, which are in fluid communication with the feeding lumen 18, allow the delivery of nutritional products or medications to be distributed along an increased length of the jejunum or small bowel.

The distal end portion of the shaft 12 may further comprise an anchoring system 78 for securing the distal end 16 of the shaft 12 within the small bowel. In the particular embodiment illustrated, the anchoring system 78 comprises a plurality of outwardly and rearwardly projecting protrusions or barbs configured to engage that interior surface of the small bowel and prevent the movement of the shaft relative thereto. In an alternative embodiment, the anchoring system 78 may comprise a suture that is tied or otherwise secured to the interior surface of the small bowel. The distal portion of the shaft 12 may also be weighted so as to inhibit the migration of the distal end 16 of the shaft 12 back towards and into the stomach.

A distal wire guide port 24 is disposed near the distal end 16 of the shaft 12 and a proximal wire guide port 26 is disposed through the side wall 28 of the shaft 12. The proximal wire guide port 26 is located a relatively short distance proximally from the distal wire guide port 24 and a substantially greater distance distally from the proximal end 14 of the shaft 12. In one embodiment of the invention, the proximal wire guide port 26 is spaced approximately 5-10 cm from the distal wire guide port 24. A wire guide lumen 22 extends between the distal wire guide port 24 and the proximal wire guide port 26, and is sized to accommodate a wire guide 30 therethrough. The wire guide lumen 30 forms a coupling region 20 for coupling the short wire PEG-J tube 60 to the wire guide 30 and has a length that is substantially less than the overall length of the shaft 12. In the embodiment of the invention illustrated, the distal wire guide port 24 comprises the distal feeding port 74, the proximal port 26 comprises one of the feeding openings 76, and the wire guide lumen 22 comprises a portion of the feeding lumen 18. In other words, the wire guide lumen 22 is formed by the distal portion of the feeding lumen 18 and feeding port/openings 74, 76. In an alternative embodiment, the wire guide lumen 22 and wire guide ports 24, 26 are separate from the feeding lumen 18 and feeding port/openings 74, 76.

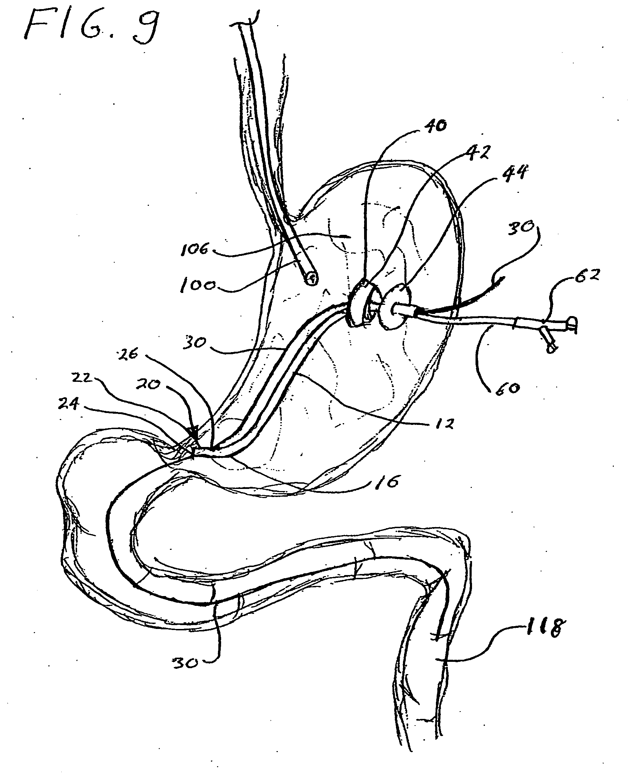

The short wire PEG-J tube 60 is introduced within the patient with the aid of a wire guide that is passed through the previously placed PEG feeding tube 40 and into the jejunum or small bowel of the patient. In this procedure, known as Percutaneous Endoscopic Jejunostomy (PEJ or PEG-J), an endoscope is first introduced (or reintroduced) into the stomach of the patient. As illustrated in Fig. 8, the distal end of a wire guide 30 is then passed through the previously placed PEG feeding tube 40 and into the stomach cavity 106, whereby it is grasped by a snare or forceps 108 deployed from the working channel of the endoscope 100. Once the distal end of the wire guide 30 is captured, the snare or forceps 108 is manipulated so as to push the distal end of the wire guide 30 into the small bowel or jejunum 118 as far as possible (see Fig. 9). Once the wire guide 30 is properly positioned, the snare or forceps 108 is withdrawn from the small bowel so that it will not interfere with the placement of the short wire PEG-J feeding tube 60 therein.

With reference to Fig. 9, the portion of the wire guide 30 extending out though the previously placed PEG feeding tube 40 is then grasped and held stationary as the short wire PEG-J tube 60 is coupled thereto. More specifically, the proximal end of the wire guide 30 is inserted in through the distal wire guide port 24, along the wire guide lumen 22, and out through proximal wire guide port 26 of the short wire PEG-J feeding tube 60. The portion of the wire guide 30 extending out though the proximal wire guide port 26 is then grasped and held stationary by the physician as the short wire PEG-J tube 60 is advanced along the wire guide 30 and into the patient through the previously placed PEG tube 40. Because the short wire PEG-J tube 60 is only coupled to the wire guide 30 for a relatively short length, as compared to the overall length of the shaft 12, the length of wire guide 30 extending out of the patient is significantly less than the overall length of the short wire PEG-J tube 60. More specifically, the portion of the wire guide 30 extending out of the previously placed PEG feeding tube 40 only needs to be slightly longer than the length of the feeding tube coupling region 20 to allow the physician to maintain control of the wire guide 30 as the feeding tube 60 is being coupled thereto. For example, if the feeding tube coupling region 20 is 6 cm in length, then the length of wire guide 30 extending out of the previously placed PEG feeding tube 40 could be as short as 10 cm. As a consequence, a much shorter wire 30 can be utilized than would be required for the introduction of a standard PEG-J tube having a wire guide lumen extending through the entire length thereof.

The short wire PEG-J feeding tube is further advanced until the distal end 16 of the shaft 12 is pushed as far into the small bowel or jejunum 118 as possible. If the short wire PEG-J feeding tube includes an anchoring system 78 (see Fig. 2), then the anchoring system 78 is secured to the inside surface of the small bowel. The wire guide 30 is then removed and the proximal end 14 of the short wire PEG-J feeding tube 60 is secured to the PEG feeding tube 40. An adapter compatible with standardized feeding pumps and syringes, if required, is then attached to the connector fitting 62. Once the short wire PEG-J feeding tube 60 is in position and secured, the endoscope 100 is withdrawn to complete the procedure. Other aspects of the procedure for the final positioning and placement of the short wire PEG-J tube 60 not described herein are similar to the procedure used for final positioning and placement of a standard PEG-J tube.

Because the wire guide 30 does not pass through a substantial portion of the shaft 12 for the short wire PEG-J feeding tube 60, the use of a support or stiffening stylet may be desirable during introduction and placement. More specifically, a support stylet (not shown) may be inserted into the feeding lumen 18 of the shaft 12 so as to provide additional stiffness and prevent the shaft 12 from bending excessively or kinking as the short wire PEG-J feeding tube 60 is pushed into final position and frictional resistance is encountered by the distal end 16 of the shaft 12. The support stylet may comprise an elongate member, such as second wire guide, that is inserted into the feeding lumen 18 from the proximal end 14 of the device. Once the short wire PEG-J feeding tube 60 is in final position, the support stylet is removed from the feeding lumen 18. In an alternative embodiment, the shaft 12 of the feeding tube may comprise a support or stiffening member (not shown) permanently embedded in or along the wall of the shaft 12. In this alternative embodiment, the support member remains as a component of the shaft after final placement of the short wire PEG-J feeding tube 60.

The wire guide 30 for use in the above described procedures for placement of a short wire PEG or PEG-J feeding tube comprises a length that is substantially shorter than the length of a wire guide that would be required for placement of a standard PEG or PEG-J feeding tube having a full length wire guide lumen. For example, the length of the wire guide 30 needed for placement of a short wire PEG feeding tube according the present invention could be 100 cm or less, and the length of the wire guide needed for placement of a short wire PEG-J feeding tube according the present invention could likewise be 100 cm or less. Because the length of the wire guide is relatively short, it may be desirable to include a bumper or stop (for example, a 1 cm diameter disk) on the proximal end of the wire guide shaft. The bumper would prevent the proximal end from being inadvertently pulled into the patient during initial placement of the wire guide 30.

The wire guide 30 may be made of any suitable maternal such as PEEK, polyvinyl chloride (PVC), polyimide, polyimide reinforced with a stainless steel braid, polyurethane, nylon, metal tubing such as nitinol or stainless steel, and the like. The wire guide 30 may also be formed as a coil or a solid-core wire guide. In one exemplary embodiment, the wire guide 30 is 75 cm in length, wherein the distal 15 cm of the wire guide 30 comprises a coated wire of increased stiffness, the proximal 10 cm comprises a non-hydrophilic coating, and the middle 50 cm comprises a hydrophilic coating to reduce frictional forces along this portion of the shaft. However, it should be appreciated that the composition and structure of the wire guide 30 is not critical to the present invention.

While there have been described what are presently believed to be the preferred embodiments of the invention, those skilled in the art will realize that changes and modifications may be made thereto. It is to be understood that the invention can be carried out by specifically different equipment and devices, and that various modifications, both as to the equipment details and operating procedures, can be accomplished without departing from the scope of the invention itself.

## Claims

1. A feeding tube apparatus (10) for placement within the gastrointestinal tract of a patient comprising an elongate shaft (12) having a distal end (16), a proximal end (14), and a feeding lumen (18) extending through at least a portion of the shaft, wherein the shaft comprises a coupling region (20) for coupling the feeding tube to a wire guide (30), the coupling region (20) being disposed near the distal end of the shaft and having a length that is substantially less than an overall length of the shaft, wherein the coupling region (20) is configured to allow the feeding tube apparatus (10) to be slidably advanced along a stationary wire guide (30) extending through the gastrointestinal tract of the patient, wherein the coupling region comprises a wire guide lumen (22) extending through at least a portion of the shaft, the wire guide lumen being sized to accommodate a wire guide extending therethrough, and wherein the wire guide lumen extends between a distal port (24) near the distal end of the shaft and a proximal port (26) spaced distally from the proximal end of the shaft.

2. The feeding tube apparatus according to claim 1 wherein the distal port (24) is disposed in the distal end of the shaft and the proximal port extends through a side wall of the shaft, further wherein the proximal port (26) is located a substantially greater distance from the proximal end of the shaft than from the distal end of the shaft.

3. The feeding tube apparatus according to claim 1 wherein the wire guide lumen (22) comprises a portion of the feeding lumen.

4. The feeding tube apparatus according to claim 1 wherein the wire guide lumen (22) is separate and spaced apart from the feeding lumen.

5. The feeding tube apparatus according to claim 1 wherein the coupling region comprises a wire guide tube attached to a side wall of the shaft, the wire guide tube being adapted to movably engage a wire guide.

6. The feeding tube apparatus according to claim1 wherein the apparatus comprises a PEG feeding tube adapted for delivery of nutritional products directly into a patient's stomach, the shaft of the apparatus being adapted to extend percutaneously through the patient's abdominal and stomach walls, the apparatus further comprising an internal bolster attached to the proximal end of the shaft and an external bolster attached to the shaft distally of the internal bolster, the internal bolster being adapted to engage an interior wall of the patient's stomach, and the external bolster being adapted to engage an exterior wall of the patient's abdomen.

7. The feeding tube apparatus according to claim 6 wherein the shaft comprises a proximal feeding tube portion and a distal lead catheter portion, the lead catheter portion being tapered to facilitate ingress of the apparatus into the patient's gastrointestinal tract and out through the patient's abdominal and stomach walls, the lead catheter portion being removable after the shaft of the apparatus is extended percutaneously through the patient's abdominal and stomach walls.

8. The feeding tube apparatus according to claim 6 wherein the wire guide lumen extends between a distal port disposed in a distal end of the lead catheter portion and a proximal port spaced proximally of the distal port, the proximal port being located a substantially greater distance from the proximal end of the shaft than from the distal end of the shaft.

9. The feeding tube apparatus according to claim 1 wherein the apparatus comprises a PEG-J feeding tube adapted for delivery of nutritional products directly into a patient's jejunum, a proximal portion of the shaft being adapted to extend through a PEG feeding tube percutanteously placed through the patient's abdominal and stomach walls, and a distal portion of the shaft being adapted to extent into the patient's jejunum, the apparatus further comprising a connector fitting attached to the proximal end of the shaft, the connector fitting being adapted to engage a feeding lumen of the PEG feeding tube.

10. The feeding tube apparatus according to claim 9 wherein the wire guide lumen extends between a distal port disposed in a distal end of the shaft and a proximal port disposed through a sidewall of the shaft, the proximal port being located a substantially greater distance from the proximal end of the shaft than from the distal end of the shaft.

11. The feeding tube apparatus according to claim 1 further comprising a wire guide extending through the coupling region of the shaft, the coupling region of the shaft being axially movable along the wire guide.

12. The feeding tube apparatus according to claim 11 wherein wire guide comprises a length that is not substantially greater than an overall length of the shaft of the feeding tube apparatus.

13. The feeding tube apparatus according to Claim 12 wherein the length of the wire guide is less than 1.5 times the overall length of the shaft of the feeding tube apparatus.

## Patentansprüche

1. Ernährungssonden-Vorrichtung (10) zur Platzierung innerhalb des gastrointestinalen Trakts eines Patienten, die einen länglichen Schaft (12) mit einem distalen Ende (16), einem proximalen Ende (14) und einem Ernährungssondenlumen (18) aufweist, das sich durch mindestens einen Abschnitt des Schaftes erstreckt, wobei der Schaft einen Kopplungsbereich (20) zum Ankoppeln der Ernährungssonde an einen Führungsdraht (30) aufweist, der Kopplungsbereich (20) in der Nähe des distalen Endes des Schaftes angeordnet ist und eine Länge aufweist, die wesentlich geringer als die Gesamtlänge des Schaftes ist, wobei der Kopplungsbereich (20) derart ausgebildet ist, dass er der Ernährungssonden-Vorrichtung (10) ermöglicht, gleitend entlang eines unbeweglichen Führungsdrahts (30), der sich durch den gastrointestinalen Trakt des Patienten erstreckt, vorgeschoben zu werden, wobei der Kopplungsbereich ein Führungsdrahtlumen (22) aufweist, das sich durch mindestens einen Abschnitt des Schaftes erstreckt, wobei das Führungsdrahtlumen derart dimensioniert ist, dass es einen Führungsdraht aufnimmt, der sich dort hindurch erstreckt, und wobei sich das Führungsdrahtlumen zwischen einer distalen Durchlassöffnung (24), die in der Nähe des distalen Endes des Schaftes liegt, und einer proximalen Durchlassöffnung (26) erstreckt, die von dem proximalen Ende des Schaftes distal beabstandet angeordnet ist.

2. Ernährungssonden-Vorrichtung nach Anspruch 1, wobei die distale Durchlassöffnung (24) in dem distalen Ende des Schaftes angeordnet ist und sich die proximale Durchlassöffnung durch eine Seitenwand des Schaftes erstreckt, wobei ferner die proximale Durchlassöffnung (26) in einem wesentlich größeren Abstand von dem proximalen Ende des Schaftes als von dem distalen Ende des Schaftes positioniert ist.

3. Ernährungssonden-Vorrichtung nach Anspruch 1, wobei das Führungsdrahtlumen (22) einen Abschnitt des Ernährungssondenlumens umfasst.

4. Ernährungssonden-Vorrichtung nach Anspruch 1, wobei das Führungsdrahtlumen (22) separat vorliegt und von dem Ernährungssondenlumen räumlich beabstandet ist.

5. Ernährungssonden-Vorrichtung nach Anspruch 1, wobei der Kopplungsbereich einen Führungsdrahtschlauch aufweist, der an einer Seitenwand des Schaftes befestigt ist, wobei der Führungsdrahtschlauch angepasst ist, mit einem Führungsdraht beweglich im Eingriff zu stehen.

6. Ernährungssonden-Vorrichtung nach Anspruch 1, wobei die Vorrichtung eine PEG-Ernährungssonde aufweist, die für die Zuführung von Ernährungsprodukten unmittelbar in den Magen eines Patienten angepasst ist, wobei der Schaft der Vorrichtung angepasst ist, sich perkutan durch die Bauchdecke und die Magenwand des Patienten hindurch zu erstrecken, wobei die Vorrichtung ferner ein internes Polster aufweist, welches an dem proximalen Ende des Schaftes angebracht ist, und ein externes Polster, welches an dem Schaft distal zu dem internen Polster angebracht ist, wobei das interne Polster angepasst ist, mit einer Innenseite der Magenwand des Patienten in Eingriff zu stehen und das externe Polster angepasst ist, mit einer Außenseite der Bauchdecke des Patienten in Eingriff zu stehen.

7. Ernährungssonden-Vorrichtung nach Anspruch 6, wobei der Schaft einen proximalen Ernährungssonden-Abschnitt und einen distalen Führungskatheter-Abschnitt aufweist, wobei der Führungskatheter-Abschnitt verjüngt ist, um den Eintritt der Vorrichtung in den gastrointestinalen Trakt des Patienten und den Austritt durch die Bauchdecke und die Magenwand des Patienten zu unterstützen, wobei der Führungskatheter-Abschnitt abnehmbar ist, nachdem der Schaft der Vorrichtung perkutan durch die Bauchdecke und die Magenwand des Patienten hindurchgetreten ist.

8. Ernährungssonden-Vorrichtung nach Anspruch 6, wobei sich das Führungsdrahtlumen zwischen einer distalen Durchlassöffnung, die an dem distalen Ende des Führungskatheter-Abschnitts angeordnet ist, und einer proximalen Durchlassöffnung erstreckt, die proximal zu der distalen Durchlassöffnung beabstandet ist, wobei die proximale Durchlassöffnung in einem wesentlich größeren Abstand zu dem proximalen Ende des Schaftes als zu dem distalen Ende des Schaftes positioniert ist.

9. Ernährungssonden-Vorrichtung nach Anspruch 1, wobei die Vorrichtung eine PEG-J-Ernährungssonde aufweist, die für die Zuführung von Ernährungsprodukten unmittelbar in das Jejunum eines Patienten angepasst ist, wobei ein proximaler Abschnitt des Schaftes angepasst ist, sich durch eine PEG-Ernährungssonde zu erstrecken, die perkutan durch die Bauchdecke und die Magenwand des Patienten platziert wurde, und ein distaler Abschnitt des Schaftes angepasst ist, sich in das Jejunum des Patienten hinein zu erstrecken, die Vorrichtung ferner einen Verbindungsstutzen aufweist, der an dem proximalen Ende des Schaftes angebracht ist, wobei der Verbindungsstutzen angepasst ist, mit einem Ernährungssondenlumen der PEG-Ernährungssonde in Eingriff zu treten.

10. Ernährungssonden-Vorrichtung nach Anspruch 9, wobei sich das Führungsdrahtlumen zwischen einer distalen Durchlassöffnung, die an dem distalen Ende des Schaftes angeordnet ist, und einer proximalen Durchlassöffnung erstreckt, die durch eine Seitenwand des Schaftes angebracht ist, wobei die proximale Durchlassöffnung in einem wesentlich größeren Abstand zu dem proximalen Ende des Schaftes als zu dem distalen Ende des Schaftes positioniert ist.

11. Ernährungssonden-Vorrichtung nach Anspruch 1, die ferner einen Führungsdraht aufweist, der sich durch den Kopplungsbereich des Schaftes hindurch erstreckt, wobei der Kopplungsbereich des Schafts axial entlang des Führungsdrahts bewegbar ist.

12. Ernährungssonden-Vorrichtung nach Anspruch 11, wobei der Führungsdraht eine Länge aufweist, die nicht wesentlich größer ist als eine Gesamtlänge des Schaftes der Ernährungssonden-Vorrichtung.

13. Ernährungssonden-Vorrichtung nach Anspruch 12, wobei die Länge des Drahtes weniger als das 1,5-fache der Gesamtlänge des Schaftes der Ernährungssonden-Vorrichtung beträgt.

## Revendications

1. Appareil (10) de sonde d'alimentation destiné à être positionné à l'intérieur du tractus gastrointestinal d'un patient comportant une tige (12) allongée présentant une extrémité (16) distale, une extrémité (14) proximale, et une lumière (18) d'alimentation traversant au moins une section de la tige, dans lequel la tige comporte une région (20) d'accouplement pour accoupler la sonde d'alimentation à un guide-fil (30), la région (20) d'accouplement étant disposée près de l'extrémité distale de la tige et ayant une longueur qui est sensiblement inférieure à la longueur totale de la tige, dans lequel la région (20) d'accouplement est configurée pour permettre à l'appareil (10) de sonde d'alimentation d'être acheminé de manière coulissante le long d'un guide-fil (30) stationnaire s'étendant dans le tractus gastrointestinal du patient, dans lequel la région d'accouplement comporte une lumière (22) de guide-fil traversant au moins une section de la tige, la lumière de guide-fil étant dimensionnée pour loger un guide fil la traversant, et dans lequel la lumière de guide-fil s'étend entre un orifice (24) distal près de l'extrémité distale de la tige et un orifice (26) proximal espacé de manière distale de l'extrémité proximale de la tige.

2. Appareil de sonde d'alimentation selon la revendication 1 dans lequel l'orifice (24) distal est disposé dans l'extrémité distale de la tige et l'orifice proximal traverse une paroi latérale de la tige, dans lequel en outre l'orifice (26) proximal est situé à une distance sensiblement plus grande de l'extrémité proximale de la tige que de l'extrémité distale de la tige.

3. Appareil de sonde d'alimentation selon la revendication 1 dans lequel la lumière (22) de guide-fil comporte une section de lumière d'alimentation.

4. Appareil de sonde d'alimentation selon la revendication 1 dans lequel la lumière (22) de guide-fil est séparée de la lumière d'alimentation et espacée par rapport à cette dernière.

5. Appareil de sonde d'alimentation selon la revendication 1 dans lequel la région d'accouplement comporte une sonde de guide-fil fixée à une paroi latérale de la tige, la sonde de guide-fil étant conçue pour venir en contact de manière mobile avec un guide-fil.

6. Appareil de sonde d'alimentation selon la revendication 1 dans lequel l'appareil comporte une sonde d'alimentation PEG conçue pour administrer des produits nutritionnels directement dans l'estomac d'un patient, la tige de l'appareil étant conçue pour traverser de manière percutanée les parois de l'abdomen et de l'estomac du patient, l'appareil comportant en outre un traversin interne fixé à l'extrémité proximale de la tige et un traversin externe fixé à la tige de manière distale par rapport au traversin interne, le traversin interne étant conçu pour venir en contact avec une paroi interne de l'estomac du patient, et le traversin externe étant conçu pour venir en contact avec une paroi externe de l'abdomen du patient.

7. Appareil de sonde d'alimentation selon la revendication 6 dans lequel la tige comporte une section de sonde d'alimentation proximale et une section distale de cathéter d'avance, la section de cathéter d'avance étant conique pour faciliter l'entrée de l'appareil dans le tractus gastro-intestinal du patient et sa sortie à travers les parois de l' abdomen et de l'estomac du patient, la section de cathéter d'avance étant détachable une fois la tige de l'appareil étendue de manière percutanée à travers les parois de l'abdomen et de l'estomac du patient.

8. Appareil de sonde d'alimentation selon la revendication 6 dans lequel la lumière de guide-fil s'étend entre un orifice distal disposé dans une extrémité distale de la section de cathéter d'avance et un orifice proximal espacé de manière proximale par rapport à l'orifice distal, l'orifice proximal étant situé à une distance sensiblement plus grande de l'extrémité proximale de la tige que de l'extrémité distale de la tige.

9. Appareil de sonde d'alimentation selon la revendication 1 dans lequel l'appareil comporte une sonde d'alimentation PEG-J conçue pour administrer des produits nutritionnels directement dans le jéjunum d'un patient, une section proximale de la tige étant conçue pour s'étendre dans une sonde d'alimentation PEG placée de manière percutanée à travers les parois de l'estomac et de l'abdomen du patient, et une section distale de la tige étant conçue pour s'étendre dans le jéjunum du patient, l'appareil comportant en outre un embout de raccordement fixé à l'extrémité proximale de la tige, l'embout de raccordement étant conçu pour venir en contact avec une lumière d'alimentation de la sonde d'alimentation PEG.

10. Appareil de sonde d'alimentation selon la revendication 9 dans lequel la lumière de guide-fil s'étend entre un orifice distal disposé dans une extrémité distale de la tige et un orifice proximal disposé à travers une paroi latérale de la tige, l'orifice proximal étant situé à une distance sensiblement plus grande de l'extrémité proximale de la tige que de l'extrémité distale de la tige.

11. Appareil de sonde d'alimentation selon la revendication 1 comportant en outre un guide-fil traversant la région d'accouplement de la tige, la région d'accouplement de la tige étant mobile dans le sens axial le long du guide-fil.

12. Appareil de sonde d'alimentation selon la revendication 11 dans lequel le guide-fil comporte une longueur qui n'est sensiblement pas supérieure à la longueur totale de la tige de l'appareil de sonde d'alimentation.

13. Appareil de sonde d'alimentation selon la revendication 12 dans lequel la longueur du guide-fil est inférieure à 1,5 fois la longueur totale de la tige de l'appareil de sonde d'alimentation.
